# NOUVEAU FASCICULE DE BREVET EUROPEEN

(11) **EP 0 515 539 B2**
(45) Date de publication et mention de la décision concernant l'opposition: **27.08.1997**
(45) Mention de la délivrance du brevet: 05.10.1994
(21) Numéro de dépôt: 91905043.5
(22) Date de dépôt: 15.02.1991
(51) Int. Cl.: A61B 17/22, A61B 1/04, F28G 15/08, B08B 7/00

(54) **SONDE MULTICANALAIRE**
SONDE MIT MEHREREN KANÄLEN
MULTI-CHANNEL PROBE

(30) Priorité: 16.02.1990 FR 9002219; 19.02.1990 FR 9001981
(43) Date de publication de la demande: 02.12.1992
(73) Titulaire: AMIEL, Jean, F-06300 Nice (FR)
(72) Inventeur: AMIEL, Jean, F-06300 Nice (FR)
(74) Mandataire: Portal, Gérard
(86) Numéro de dépôt international: FR9100124
(87) Numéro de publication internationale: WO9111963

(56) Documents cités:
- EP-A- 0 266 928
- EP-A- 0 350 021
- EP-A- 0 370 115
- WO-A-89/12479
- US-A- 4 800 876
- US-A- 4 887 600

## Description

Sonde multicanalaire à au moins trois canaux longitudinaux indépendants utilisable pour la destruction d'obstacles lithiasiques par voie endoscopique ainsi que pour la destruction de dépôts minéraux dans des conduits.

### DOMAINE D'APPLICATION DE L'INVENTION

La présente invention concerne essentiellement une sonde multicanalaire à au moins trois canaux longitudinaux indépendants utilisable pour la destruction d'obstacles lithiasiques par voie endoscopique ainsi que pour la destruction de dépôts minéraux dans des conduits.

Cette sonde multicanalaire peut être utilisée pour réaliser un diagnostic ainsi que le traitement endoscopique d'obstacles lithiasiques, plus particulièrement des obstacles lithiasiques rencontrés en urologie et gastro-entérologie.

La sonde multicanalaire peut être également utilisée dans des domaines autres que le domaine médical, Iorsqu'il est nécessaire de détruire des dépôts minéraux déposés dans des conduits et dont l'accès doit être fait par voie endoluminale, cette destruction étant commandée et réalisée à distance.

### ARRIERE PLAN TECHNOLOGIQUE

On connaît, par le document FOX US-A-4 800 876, une sonde endoscopique comprenant un élément tubulaire à l'intérieur duquel sont disposées de manière fixe des fibres optiques de traitement par rayonnement laser et des fibres optiques d'observation.

De même, le document WO-A-89/12479 OPTIMED décrit un cathéter pour l'angioplastie comprenant une fibre optique solidarisée de manière définitive audit dispositif et comprenant un ballonnet augmentant le diamètre extérieur du cathéter.

Par ailleurs, il est également connu, par le document EP-A-0 350 021, la destruction de contaminant de surface par une irradiation directe par une source de haute énergie pouvant comprendre un laser pulsé.

Les solutions proposées ne donnent pas entière satisfaction. En particulier, dans le cadre du domaine médical pour la destruction d'obstacles lithiasiques, en particulier rencontrés en urologie et gastro-entérologie, les dispositifs endoscopiques existant jusqu'à présent sont constitués d'un corps rigide, semi-rigide ou flexible dans lequel est incorporé de façon inamovible le système optique et qui comprend généralement un canal opérateur servant à véhiculer la fibre laser et le liquide de rinçage nécessaires au bon fonctionnement de l'ensemble.

Après la destruction des obstacles lithiasiques, la sonde endoscopique de traitement est retirée et il est alors nécessaire de mettre en place temporairement dans la voie excrétrice urinaire une sonde urétérale de drainage. Ceci implique une procédure compliquée, qui augmente considérablement le temps nécessaire à l'ensemble de l'intervention, ce qui la rend incompatible avec une absence d'anesthésie et par conséquent, nécessite une hospitalisation.

En outre, les dispositifs endoscopiques actuels sont très coûteux et ne peuvent donc pas être à usage unique, ce qui pose le problème de leur stérilisation; et notamment la stérilisation du canal opérateur.

Egalement, en raison de la finesse nécessaire à ces dispositifs endoscopiques, ils sont d'une grande fragilité, ce qui rend leur durée de vie relativement courte et leur entretien onéreux.

Le document EP-A-0 266 928 décrit une sonde multicanalaire dans laquelle les quarte canaux ne traversent pas complètement d'une extremité à l'autre.

### RESUME DE L'INVENTION

La présente invention a donc pour but de résoudre le nouveau problème technique consistant en la fourniture d'une solution permettant de réaliser la destruction d'obstacles lithiasiques raccourcissant le temps nécessaire à l'ensemble de l'intervention et évitant en conséquence une anesthésie générale.

La présente invention a encore pour but de résoudre le nouveau problème technique consistant en la fourniture d'une solution permettant de réaliser la destruction d'obstacles lithiasiques à l'aide d'un dispositif d'un plus faible coût, moins fragile, et permettant une stérilisation aisée voire un usage unique.

La présente invention a encore pour but de résoudre le nouveau problème technique consistant en la fourniture d'une solution permettant de réaliser la destruction d'obstacles Iithiasiques à l'aide d'un dispositif endoscopique, qui puisse également être utilisé à titre temporaire pour réaliser un drainage après destruction desdits obstacles lithiasiques.

La présente invention a également pour but de résoudre le nouveau problème technique consistant en la fourniture d'une solution qui permette encore de réaliser la destruction de dépôts minéraux dans des conduits, dont l'accès doit être réalisé par voie endoluminale et le traitement de destruction commandé et réalisé à distance en raison de l'inaccessibilité.

Tous ces problèmes sont résolus par la présente invention d'une manière simple, peu coûteuse, fiable, utilisable industriellement.

A cet effet, selon un premier aspect, la présente invention fournit une sonde multicanalaire comprenant un élément tubulaire creux définissant une extrémité distale et une extrémité proximale, elle est réalisée en un matériau auto-portant souple, permettant de se plier ou s'incurver, ledit élément tubulaire est totalement creux, et sub-divisé en au moins quatre canaux longitudinaux indépendants par au moins trois cloisons de refend disposées longitudinalement et sensiblement parallèlement à l'axe de symétrie de l'élément tubulaire et traversent complètement l'élément tubulaire de l'extremité proximale à l'extremité distale; la surface externe de l'élément tubulaire est cylindrique et lisse sur sensiblement toute sa longueur.

Selon une variante de réalisation avantageuse, la sonde multicanalaire est une sonde multicanalaire endocavitaire pour le diagnostic et/ou le traitement endoscopique des obstacles lithiasiques, tels que ceux rencontrés en urologie et gastro-entérologie.

Selon un mode de réalisation avantageux, la sonde multicanalaire est caractérisée en ce que ladite sonde est à usage unique.

Selon une autre variante de réalisation particulière, l'extrémité distale de l'élément tubulaire est effilée.

Selon un autre mode de réalisation avantageux de l'invention, deux canaux dits petits canaux présentent un diamètre plus petit que le canal ou les canaux restants dit grand canal ou grands canaux.

Selon un autre mode de réalisation avantageux, deux canaux dits petits canaux sont de diamètre plus petit que les deux canaux restants dits grands canaux, la disposition des cloisons de refend réalisant de préférence une structure symétrique de la sonde dans au moins un plan de symétrie.

Selon une variante de réalisation particulière, une cloison de refend est disposée selon le diamètre et traverse complètement l'élément tubulaire, et deux cloisons de refend s'étendent de cette cloison de refend diamétrale pour définir les deux petits canaux précités.

Selon une variante de réalisation particulière, les deux petits canaux présentent un diamètre, qui est de l'ordre de la moitié du diamètre du grand canal ou des deux grands canaux précités.

Selon une variante de réalisation particulière, un canal, dit canal de traitement, en particulier un petit canal, est destiné à recevoir provisoirement une fibre optique adaptée pour recevoir un rayonnement laser et en particulier un rayonnement d'un laser pulsé.

Selon une autre variante de réalisation particulière, un canal précité, dit canal d'observation, en particulier un grand canal, est destiné à recevoir provisoirement une fibre optique adaptée pour recevoir un rayonnement optique d'observation et d'éclairage de la zone extérieure à l'élément tubulaire au voisinage de l'extrémité distale, en particulier pour former une image vidéo.

Selon encore une autre variante de réalisation particulière, un canal dit canal d'irrigation, en particulier un petit canal, reçoit un milieu liquide d'irrigation ou anesthésique (comme la Xylocaïne) de la zone extérieure à l'élément tubulaire au voisinage de l'extrémité distale.

Selon un mode de réalisation particulièrement avantageux, la sonde multicanalaire précitée est caractérisée en ce que cette sonde multicanalaire comprend quatre canaux indépendants, par la présence d'au moins trois cloisons de refend disposées longitudinalement et sensiblement parallèlement à l'axe de symétrie de ladite sonde, un premier canal de traitement destiné à recevoir provisoirement une fibre optique adaptée à recevoir un rayonnement laser, en particulier un rayonnement laser pulsé, un deuxième canal d'observation destiné à recevoir une fibre optique adaptée pour permettre au moins l'observation de la zone extérieure au voisinage de l'extrémité distale, un troisième canal d'irrigation destiné à recevoir un milieu liquide d'irrigation de la zone extérieure à l'extrémité distale, et un quatrième canal d'insertion destiné à recevoir un fil guide ou leader permettant de faciliter l'insertion par guidage de la sonde multicanalaire dans un lumen du corps.

Selon encore une autre variante de réalisation particulière, l'extrémité proximale précitée est pour-vue d'un dispositif de raccordement étanche des canaux indépendants comprenant des conduits d'alimentation, éventuellement démontable ou détachable permettant l'amenée des dispositifs à insérer dans les canaux respectifs ainsi que l'injection d'un milieu liquide d'irrigation.

Selon une autre variante de réalisation particulière, l'élément tubulaire est réalisé au moins en partie en un matériau radio-opaque.

Selon un deuxième aspect, la présente invention couvre également I'utilisation de la sonde multicanalaire précitée pour la réalisation d'un appareil pour la destruction d'obstacles lithiasiques par ondes de choc, notamment à l'aide d'un laser pulsé, en particulier à colorant.

Selon un troisième aspect, la présente invention couvre également I'utilisation de la sonde multicanalaire précitée pour réaliser un appareil pour le traitement de dépôts minéraux dans des conduits, par exemple en milieu hostile comme les structures irradiées, des boues à l'intérieur d'organes mécaniques, des dépôts minéraux dans des tubes des générateurs de vapeur de centrale de production thermique, sans limitation.

Selon un quatrième aspect, la sonde peut également être utilisée pour réaliser une chirurgie percutanée notamment du rein (non couverte elle-même par les revendications).

Selon un cinquième aspect, la sonde multicanalaire selon l'invention, comprenant un élément tubulaire creux définissant une extrémité distale et une extrémité proximale, caractérisée en ce qu'elle est réalisée en un matériau autoportant souple, permettant de se plier ou s'incurver, et en ce que ledit élément tubulaire est totalement creux, et est subdivisé en au moins quatre canaux longitudinaux indépendants par au moins trois cloisons de refend disposées longitudinalement et sensiblement parallèlement à l'axe de symétrie de l'élément tubulaire ; la surface externe de l'élément tubulaire est cylindrique et lisse sur sensiblement toute sa longueur ; est utilisée pour la mise en oeuvre d'un procédé de traitement d'obstacles lithiasiques par voie endoscopique (non couvert lui-même par les revendications). Dans le cadre de cette mise en oeuvre, on prévoit :
- au moins une fibre optique dite de traitement de dimension adaptée pour pouvoir être insérée dans au moins un desdits canaux et capable de recevoir le faisceau d'un rayonnement laser, en particulier d'un rayonnement de laser pulsé,
- au moins une fibre optique dite d'observation de dimension adaptée pour pouvoir être insérée dans au moins un autre canal et adaptée pour recevoir un rayonnement lumineux permettant l'observation d'une zone extérieure à l'élément tubulaire au voisinage de l'extrémité distale,
- des moyens d'injection d'au moins un milieu liquide d'irrigation dans au moins un canal restant;
- on introduit ladite sonde multicanalaire dans un lumen du corps où est supposé se trouver un obstacle lithiasique jusqu'à ce que l'extrémité distale vienne en contact avec ledit obstacle lithiasique,
- on introduit la fibre optique adaptée à recevoir un rayonnement laser dans son canal associé,
- on introduit la fibre optique adaptée à la transmission d'un rayonnement lumineux d'observation dans son canal associé,
- lorsque cela est souhaité, on injecte dans le canal d'irrigation un milieu liquide d'irrigation grâce audit moyen d'injection de liquide d'irrigation,
- on procède à la destruction de l'obstacle lithiasique à l'aide du rayonnement laser transmis par la fibre optique adaptée à recevoir le rayonnement laser, sous contrôle visuel d'observation de la zone de traitement extérieure au voisinage de l'extrémité distale grâce au rayonnement lumineux transmis par la fibre optique d'observation,
- en fin de traitement de destruction de l'obstacle lithiasique, on retire la fibre optique de traitement recevant le rayonnement laser et la fibre optique d'observation, et on arrête l'injection de milieu liquide d'irrigation.

Selon un mode de réalisation préféré de ce procédé, celui-ci est caractérisé en ce qu'on laisse la sonde multicanalaire en position dans ledit lumen du corps pendant une période de temps prédéterminée de manière à réaliser un drainage du lumen du corps, éventuellement après démontage ou détachement d'un dispositif de raccordement étanche utilisé pour le traitement.

Selon un mode de réalisation avantageux du procédé (non couvert lui-même par les revendications), celui-ci est caractérisé en ce la sonde multicanalaire comprenant quatre canaux indépendants définis par au moins trois cloisons de refend disposées longitudinalement et parallèlement à l'axe de symétrie de l'élément tubulaire, un premier canal de traitement est destiné à recevoir une fibre optique de traitement, adaptée pour recevoir un rayonnement laser, en particulier un rayonnement laser pulsé, un second canal d'observation est destiné à recevoir une fibre optique d'observation adaptée à la transmission d'un rayonnement lumineux d'observation, un troisième canal d'irrigation est destiné à recevoir un milieu liquide d'irrigation, et un quatrième canal de guidage est destiné à recevoir un fil guide ou leader souple,
- on introduit dans le lumen du corps supposé contenir un obstacle lithiasique, le fil guide précité, en particulier grâce à un cytoscope,
- on introduit ensuite la sonde multicanalaire dans le lumen du corps en introduisant l'extrémité libre apparente du fil guide dans le quatrième canal de guidage adapté à recevoir le fil guide en faisant coulisser la sonde multicanalaire sur le fil guide jusqu'à ce que l'extrémité distale de la sonde multicanalaire arrive au contact de l'obstacle lithiasique,
- on introduit la fibre optique de traitement dans le premier canalde traitement en la faisant coulisser dans ledit premier canal de traitement jusqu'à ce qu'elle arrive au contact de l'obstacle lithiasique,
- on introduit la fibre optique d'observation dans le deuxième canal d'observations et on la fait coulisser jusqu'à ce que ladite fibre optique d'observation arrive à proximité de l'extrémité distale de ladite sonde multicanalaire,
- on procède à la destruction de l'obstacle lithiasique grâce au rayonnement laser, en particulier un rayonnement laser pulsé, sous contrôle visuel grâce à l'observation par la fibre optique d'observation transmettant le rayonnement lumineux,
- à la fin du traitement de destruction de l'obstacle lithiasique, on retire le fil guide.

Selon une variante de réalisation avantageuse du procédé, on réalise une anesthésie locale du lumen du corps en injectant dans le canal d'irrigation un agent anesthésiant au fur et à mesure du coulissement de la sonde multicanalaire sur le fil guide à l'intérieur du lumen du corps.

D'autre part, selon encore une autre variante avantageuse du procédé selon l'invention (non couvert lui-même par les revendications), en fin de traitement, après avoir retiré les fibres de traitement et d'observation, on injecte dans le canal d'irrigation un produit de contraste permettant de vérifier le positionnement correct de la sonde multicanalaire en vue d'assurer un drainage après le traitementde l'obstacle lithiasique et éventuellement on réalise un nouveau positionnement de la sonde multicanalaire par coulissement sur le fil guide, enfin, on retire le fil guide lorsqu'il est estimé que la sonde multicanalaire est en position correcte dans le lumen du corps.

Dans le cadre de l'application à la destruction de dépôts minéraux, l'invention couvre un procédé de traitement des dépôts minéraux, dans des conduits contenant lesdits dépôts minéraux, par voie endoluminale, comprenant l'emploi d'une sonde endoluminale comprenant un élément tubulaire creux comprenant une extrémité proximale et une extrémité distale de traitement, caractérisé en ce qu'on prévoit un élément tubulaire totalement creux sub-divisé en au moins quatre canaux longitudinaux indépendants par au moins trois cloisons de refend disposées longitudinalement et sensiblement parallèlement à l'axe de symétrie de l'élément tubulaire et s'étendant de l'extrémité proximale à l'extrémité distale, la surface externe de l'élément tubulaire étant cylindrique et lisse sur sensiblement toute sa longueur,
- au moins une fibre optique dite de traitement de dimension adaptée pour pouvoir être insérée dans au moins un desdits canaux et capable de recevoir le faisceau d'un rayonnement laser, en particulier d'un rayonnement de laser pulsé,
- au moins une fibre optique dite d'observation de dimension adaptée pour pouvoir être insérée dans au moins un autre canal et adaptée pour recevoir un rayonnement lumineux permettant l'observation d'une zone extérieure à l'élément tubulaire au voisinage de l'extrémité distale,
- des moyens d'injection d'au moins un milieu liquide d'irrigation dans au moins un canal restant ;
- on introduit ladite sonde multicanalaire dans un lumen du conduit où est supposé se trouver un dépôt minéral jusqu'à ce que l'extrémité distale vienne en contact avec ledit dépôt minéral,
- on introduit la fibre optique adaptée à recevoir un rayonnement laser dans son canal associé,
- on introduit la fibre optique adaptée à la transmission d'un rayonnement lumineux d'observation dans son canal associé,
- lorsque cela est souhaité, on injecte dans le canal d'irrigation un milieu liquide d'irrigation grâce audit moyen d'injection de liquide d'irrigation,
- on procède à la destruction du dépôt minéral à l'aide du rayonnement laser transmis par la fibre optique adaptée à recevoir le rayonnement laser, sous contrôle visuel d'observation de la zone de traitement extérieure au voisinage de l'extrémité distale grâce au rayonnement lumineux transmis par la fibre optique d'observation,
- en fin de traitement de destruction du dépôt minéral, on retire la fibre optique de traitement recevant le rayonnement laser et la fibre optique d'observation, et on arrête l'injection de milieu liquide d'irrigation, à l'exclusion de toute application thérapeutique et chirurgicale du procédé.

Ces dépôts minéraux peuvent être présents dans un milieu hostile comme une structure irradiée, il peut s'agir aussi de boues à l'intérieur d'organes mécaniques, de dépôts minéraux dans des tubes de générateurs de vapeur de centrale de production thermique, sans limitation.

D'autres buts, caractéristiques et avantages de l'invention apparaîtront clairement à la lumière de la description explicative qui va suivre faite en référence aux dessins annexés représentant un mode de réalisation actuellement préféré d'une sonde multicanalaire selon l'invention, donné simplement à titre d'illustration et qui ne saurait donc en aucune façon limiter la portée de l'invention.

Dans les dessins :
- la figure 1 est une vue schématique d'un mode de réalisation actuellement préféré d'une sonde multicanalaire selon la présente invention, pourvue d'un dispositif de raccordement étanche, éventuellement démontable ou détachable, permettant l'amenée de dispositifs à insérer dans les canaux respectifs ainsi que l'injection d'un milieu liquide d'irrigation,
- la figure 2 représente une vue en coupe selon la ligne de coupe II-II de la figure 1.

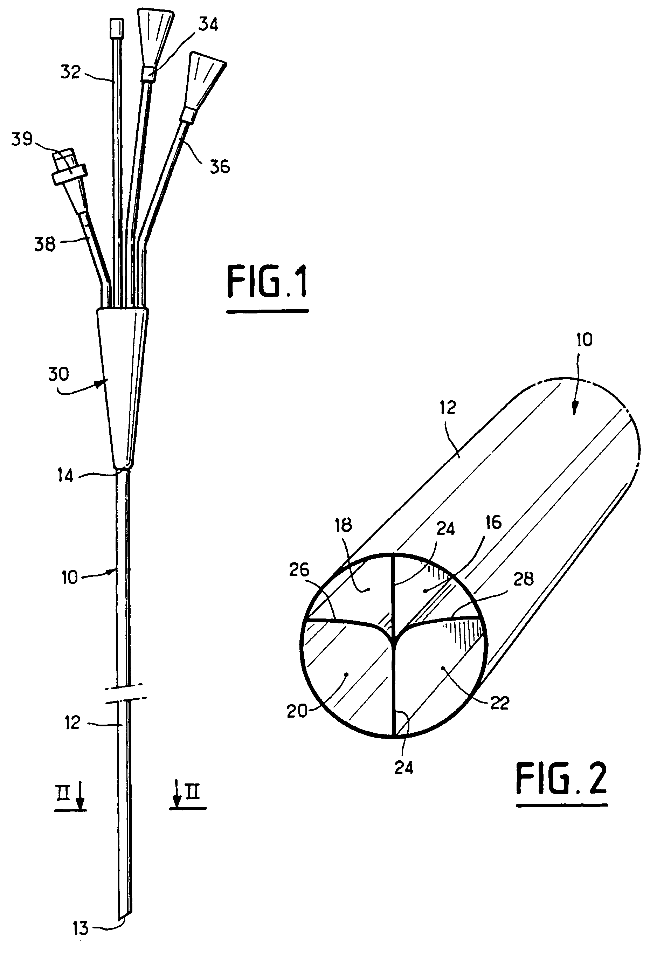

En référence aux figures 1 et 2, une sonde multicanalaire selon la présente invention est représentée par le numéro de référence général 10. Cette sonde multicanalaire 10 comprend un élément tubulaire 12 creux définissant une extrémité distale 13 et une extrémité proximale 14.

Cette sonde multicanalaire est réalisée en un matériau auto-portant souple permettant de se plier ou s'incurver. De tels matériaux auto-portants souples sont également avantageusement biocompatibles. Des exemples sont du PVC, un silicone. Cette sonde peut être rendue radio-opaque par l'introduction dans sa masse de matériaux radio-opaques. De tels matériaux radio-opaques sont bien connus à l'homme de l'art.

Selon l'invention, l'élément tubulaire creux 12 est sub-divisé en au moins trois canaux longitudinaux indépendants, c'est-à-dire non communiquants, tels que 16, 18, 20, 22 par au moins deux cloisons de refend telles que 24, 26, 28, disposées longitudinalement sensiblement parallèlement à l'axe de symétrie de l'élément tubulaire 12.

Selon un mode de réalisation préféré, tel que représenté à la figure 2, cette sonde multicanalaire 10 est caractérisée en ce que l'élément tubulaire 12 est sub-divisé en quatre canaux indépendants, référencés respectivement 16, 18, 20, 22 par au moins trois cloisons de refend respectivement référencées 24, 26, 28 disposées longitudinalement et sensiblement parallèlement à l'axe de symétrie de l'élément tubulaire 12.

Selon une variante de réalisation avantageuse, deux canaux dits petits canaux référencés ici 16, 18 présentent un diamètre plus petit que le canal ou les canaux restants dit grand canal ou grands canaux référencés ici respectivement 20, 22. De préférence, la disposition des cloisons de refend 24, 26, 28 réalise une structure symétrique de la sonde multicanalaire 10 dans au moins un plan de symétrie, comme cela est représenté clairement à la figure 2, le plan de symétrie étant le plan de la cloison de refend 24.

Selon une variante de réalisation particulière, une cloison de refend, ici la cloison de refend 24, est disposée selon un diamètre et traverse complètement l'élément tubulaire 12, et deux cloisons de refend, ici les cloisons de refend 26, 28 s'étendent de cette cloison de refend diamétrale 24 pour définir les deux petits canaux précités 16, 18.

Selon une variante de réalisation particulière avantageuse, les deux petits canaux 16, 18 ont un diamètre qui est de l'ordre de la moitié du diamètre du grand canal ou des deux grands canaux référencés 20, 22.

Un canal, dit canal de traitement, en particulier un petit canal, ici le canal 16, est destiné à recevoir provisoirement une fibre optique (non repésentée) adaptée pour recevoir un rayonnement laser et en particulier un rayonnement d'un laser pulsé. De telles fibres optiques sont bien connues à l'homme de l'art.

Un autre canal, dit canal d'observation, en particulier un grand canal tel que le canal 22 peut être avantageusement destiné à recevoir provisoirement une fibre optique ou un ensemble de fibres optiques adaptées pour former une image vidéo, bien connu à l'homme de l'art.

Selon une autre variante de réalisation particulière, un canal, en particulier un petit canal, tel que 18, est adapté pour recevoir un milieu liquide d'irrigation de la zone extérieure à l'élément tubulaire au voisinage de l'extrémité distale.

De préférence, il est également prévu un canal, en particulier un grand canal, tel que 22, pour recevoir un fil guide ou leader permettant de faciliter l'insertion par guidage de la zone multicanalaire 10 dans un lumen du corps.

Selon encore une variante de réalisation particulièrement avantageuse, la surface externe de l'élément tubulaire 12 est cylindrique et lisse sur sensiblement toute sa longueur et son extrémité distale 13 est effilée, ou entièrement conformée pour faciliter une insertion dans un lumen, comme visible à la figure 1.

Selon une autre variante de réalisation particulière, l'extrémité proximale 14 est adaptée pour recevoir un dispositif de raccordement étanche représenté par le numéro de référence général 30 permettant le raccordement étanche des canaux indépendants 16, 18, 20, 22 avec des conduits d'alimentation ou insertion respectivement référencés 32, 34, 36, 38. Le conduit 32 sert de guidage à la fibre optique adaptée à recevoir le rayonnement laser, et aboutit en conséquence ici au petit canal 16, le conduit 34 est adapté pour recevoir la fibre optique d'observation ou le groupe de fibres optiques permettant de reconstituer une image vidéo et communique avec le canal correspondant, ici le grand canal 22, le conduit 36 est adapté à recevoir le fil guide et communique ainsi avec le canal correspondant, ici le grand canal 20, et enfin le conduit 38 est adapté pour recevoir le liquide d'irrigation et communique ainsi avec le petit canal d'irrigation 18.

Cette sonde multicanalaire avec son dispositif de raccordement 30 peut être utilisée avec n'importe quel type de laser permettant de réaliser le traitement envisagé.

Les lasers préférés sont les lasers pulsés permettant de réaliser les destructions d'obstacles lithiasiques ou de dépôts minéraux par ondes de choc, comme cela est maintenant connu à l'homme de l'art. Les lasers pulsés préférés sont les lasers pulsés à colorant.

La procédure de traitement est réalisée conformément au procédé précédemment décrit.

Par ailleurs, un prototype a été réalisé selon lequel la fibre optique recevant le rayonnement laser a un diamètre d'environ 300 microns et peut être aisément insérée dans le petit canal 16, la fibre optique transmettant le rayonnement lumineux pour l'observation de la zone de traitement, ou le faisceau de fibre optique correspondant, actuellement disponible dans le commerce, a un diamètre d'environ 500 microns. Cette fibre optique ou ce faisceau de fibre optique peut être adaptée à l'extérieur du dispositif de raccordement 30 avec une lentille de visualisation de lumen. L'élément guide ou leader a en particulier un diamètre de l'ordre de 500 microns.

Ainsi, le diamètre extérieur de la sonde multicanalaire est inférieur ou égal à 7 French Charrière et est insérable dans l'uretère sans anesthésie générale. L'unité "French" est une unité habituelle de mesure des diamètres des cathéters et d'aiguilles, un French étant égal à 1/3 d'un millimètre.

Diverses variantes de réalisation sont possibles. En particulier, le conduit 38 destiné à recevoir le milieu liquide d'irrigation peut comporter une vanne d'arrêt 39. Le milieu liquide injecté dans le conduit 38 et le canal 18 peut également être de composition variable et peut au début du traitement comprendre ou être constitué d'un agent anesthésiant. La fibre optique destinée à recevoir le laser peut être remplacée par tout autre dispositif permettant de réaliser une coagulation ou une résection à la condition que son diamètre extérieur soit compatible avec le diamètre interne d'au moins un des canaux 16, 18, 20, 22. On comprend également que ces canaux sont dans certaines mesures interchangeables au niveau de la réception des éléments fonctionnels précités.

La fabrication de la sonde multicanalaire selon l'invention est très aisée à mettre en oeuvre.

On peut mettre en oeuvre la technique de fabrication par extrusion par exemple. D'autres techniques sont bien connues à l'homme de l'art.

Le mode de réalisation des figures 1 et 2 fait partie intégrante de l'invention et de la description.

## Revendications

1. Sonde multicanalaire (10) comprenant un élément tubulaire creux (12) définissant une extrémité distale (13) et une extrémité proximale (14), elle est réalisée en un matériau auto-portant souple, permettant de se plier ou s'incurver, ledit élément tubulaire (12) est totalement creux, et subdivisé en au moins quatre canaux longitudinaux indépendants (16, 18, 20, 22) par au moins trois cloisons de refend (24, 26, 28) disposées longitudinalement et sensiblement parallèlement à l'axe de symétrie de l'élément tubulaire (12) et traversant complètement l'élément tubulaire (12) de l'extremité proximale à l'extremité distale (13) ; la surface externe de l'élément tubulaire étant cylindrique et lisse sur sensiblement toute sa longueur.

2. Sonde multicanalaire selon la revendication 1, caractérisée en ce qu'il s'agit d'une sonde multicanalaire endocavitaire (10) pour le diagnostic et/ou le traitement endoscopique des obstacles lithiasiques, tels que ceux rencontrés en urologie et gastro-entérologie.

3. Sonde multicanalaire selon la revendication 1 ou 2, caractérisée en ce que ladite sonde est à usage unique.

4. Sonde multicanalaire selon l'une des revendications 1 à 3, caractérisée en ce que l'extrémité distale de l'élément tubulaire est effilée.

5. Sonde multicanalaire selon l'une des revendications 1 à 4, caractérisée en ce que deux canaux (16, 18) dits petit canaux sont de diamètre plus petit que les deux canaux restants (20, 22) dits grands canaux, la disposition des cloisons de refend (24, 26, 28) réalisant de préférence une structure symétrique de la sonde dans au moins un plan de symétrie (24).

6. Sonde multicanalaire selon l'une des revendications 1 à 5, caractérisée en ce qu'une cloison de refend (24) est disposée selon un diamètre et traverse complètement l'élément tubulaire (12), et deux cloisons de refend (26, 28) s'étendent de cette cloison de refend diamètrale (24) pour définir les deux petits canaux précités (16, 18).

7. Sonde multicanalaire selon l'une des revendications 5 ou 6, caractérisée en ce que les deux petits canaux (16, 18) ont un diamètre qui est de l'ordre de la moitié du diamètre du grand canal (20, 22) ou des deux grands canaux (20,22).

8. Sonde multicanalaire selon l'une des revendications 1 à 7, caractérisée en ce qu'un canal (16) dit canal de traitement, en particulier un petit canal, est destiné à recevoir provisoirement une fibre optique adaptée pour recevoir un rayonnement laser et en particulier un rayonnement d'un laser pulsé.

9. Sonde multicanalaire selon l'une des revendications 1 à 8, caractérisée en ce qu'un canal (22) précité dit canal d'observation, en particulier un grand canal, est destiné à recevoir provisoirement une fibre optique adaptée pour recevoir un rayonnement optique d'observation et d'éclairage de la zone extérieure à l'élément tubulaire au voisinage de l'extrémité distale.

10. Sonde multicanalaire selon l'une des revendications 1 à 8, caractérisée en ce qu'un canal (18) dit canal d'irrigation, en particulier un petit canal, reçoit un milieu liquide d'irrigation de la zone extérieure à l'élément tubulaire au voisinage de l'extrémité distale.

11. Sonde multicanalaire selon l'une des revendications 1 à 10, caractérisée en ce que cette sonde multicanalaire (10) comprend quatre canaux indépendants (16, 18, 20, 22), par la présence de trois cloisons de refend (24, 26, 28) disposées longitudinalement et sensiblement parallèlement à l'axe de symétrie de ladite sonde, un premier canal (16) de traitement destiné à recevoir provisoirement une fibre optique adaptée à recevoir un rayonnement laser, en particulier un rayonnement laser pulsé, un deuxième canal (22) d'observation destiné à recevoir une fibre optique adaptée pour permettre au moins l'observation de la zone extérieure au voisinage de l'extrémité distale, un troisième canal (18) d'irrigation destiné à recevoir un milieu liquide d'irrigation de la zone extérieure à l'extrémité distale, et un quatrième canal (20) d'insertion destiné à recevoir un fil guide ou leader permettant de faciliter l'insertion par guidage de la sonde multicanalaire dans un lumen du corps.

12. Sonde multicanalaire selon l'une des revendications 1 à 11, caractérisée en ce que l'extrémité proximale (14) précitée est pourvue d'un dispositif de raccordement étanche (30) des canaux indépendants, comprenant des conduits (32, 34, 36, 38) d'alimentation ou insertion, éventuellement démontable ou détachable, permettant l'amenée des dispositifs à insérer dans les canaux respectifs ainsi que l'injection d'un milieu liquide d'irrigation.

13. Sonde multicanalaire selon l'une des revendications 1 à 12, caractérisée en ce que l'élément tubulaire (12) est réalisé au moins en partie en un matériau radio-opaque.

14. Utilisation d'une sonde multicanalaire selon l'une des revendications 1 à 13, pour la réalisation d'un appareil pour la destruction d'obstacles lithiasiques par ondes de choc, notamment à l'aide d'un laser pulsé, en particulier à colorant.

15. Utilisation d'une sonde multicanalaire selon l'une des revendications 1 à 13, pour réaliser un appareil pour le traitement de dépôts minéraux dans des conduits, par exemple en milieu hostile comme des structures irradiées, des boues à l'intérieur d'organes mécaniques, des dépôts minéraux dans des tubes de générateurs de vapeur de centrale de production thermique.

16. Utilisation d'une sonde multicanalaire selon l'une des revendications 1 à 13, pour la réalisation d'un appareil pour une chirurgie percutanée du rein.

17. Procédé de traitement des dépôts minéraux dans des conduits contenant lesdits dépôts minéraux, par voie endoluminale, comprenant l'emploi d'une sonde endoluminale comprenant un élément tubulaire creux comprenant une extrémité proximale et une extrémité distale de traitement, caractérisé en ce qu'on prévoit un élément tubulaire totalement creux sub-divisé en au moins quatre canaux longitudinaux indépendants par au moins trois cloisons de refend disposées longitudinalement et sensiblement parallèlement à l'axe de symétrie de l'élément tubulaire et s'étendant de l'extrémité proximale à l'extrémité distale, la surface externe de l'élément tubulaire étant cylindrique et lisse sur sensiblement toute sa longueur,
- au moins une fibre optique dite de traitement de dimension adaptée pour pouvoir être insérée dans au moins un desdits canaux et capable de recevoir le faisceau d'un rayonnement laser, en particulier d'un rayonnement de laser pulsé,
- au moins une fibre optique dite d'observation de dimension adaptée pour pouvoir être insérée dans au moins un autre canal et adaptée pour recevoir un rayonnement lumineux permettant l'observation d'une zone extérieure à l'élément tubulaire au voisinage de l'extrémité distale,
- des moyens d'injection d'au moins un milieu liquide d'irrigation dans au moins un canal restant ;
- on introduit ladite sonde multicanalaire dans un lumen du conduit où est supposé se trouver un dépôt minéral jusqu'à ce que l'extrémité distale vienne en contact avec ledit dépôt minéral,
- on introduit la fibre optique adaptée à recevoir un rayonnement laser dans son canal associé,
- on introduit la fibre optique adaptée à la transmission d'un rayonnement lumineux d'observation dans son canal associé,
- lorsque cela est souhaité, on injecte dans le canal d'irrigation un milieu liquide d'irrigation grâce audit moyen d'injection de liquide d'irrigation,
- on procède à la destruction du dépôt minéral à l'aide du rayonnement laser transmis par la fibre optique adaptée à recevoir le rayonnement laser, sous contrôle visuel d'observation de la zone de traitement extérieure au voisinage de l'extrémité distale grâce au rayonnement lumineux transmis par la fibre optique d'observation,
- en fin de traitement de destruction du dépôt minéral, on retire la fibre optique de traitement recevant le rayonnement laser et la fibre optique d'observation, et on arrête I'injection de milieu liquide d'irrigation, à l'exclusion de toute application thérapeutique et chirurgicale du procédé.

## Claims

1. Multichannel probe (10) comprising a hollow tubular element (12) defining a distal end (13) and a proximal end (14), it is made of a flexible self-supporting material, enabling the tube to fold or to be curved, said tubular element (12) is totally hollow, and subdivided into at least four independent longitudinal channels (16, 18, 20, 22) by at least three internal partitions (24, 26, 28) disposed longitudinally and substantially parallel to the axis of symmetry of the tubular element (12) from the proximal end to the distal end (13); the external surface of the tubular element is cylindrical and smooth approximately through the whole of its length.

2. Multichannel probe according to claim 1, characterized in that said probe is a multichannel endocavity probe (10) for endoscopic diagnosis and/or treatment of lithiasic obstructions, such as those encountered in urology and gastroenterology.

3. Multichannel probe according to claim 1 or 2, characterized in that said probe is a single-use probe.

4. Multichannel probe according to one of claims 1 to 3, characterized in that the distal end of the tubular element is tapered.

5. Multichannel probe according to one of claims 1 to 4, characterized in that two channels (16, 18) called small channels are of smaller diameter than the two remaining channels (20, 22) called large channels, the disposition of the internal partitions (24, 26, 28) preferably constituting a probe structure which is symmetrical about at least one plane of symmetry (24).

6. Multichannel probe according to one of claims 1 to 5, characterized in that one internal partition (24) is disposed along a diameter and extends all the way across the tubular element (12), and two internal partitions (26, 28) extend from said diametrical internal partition (24) to define the two above-specified small channels (16, 18).

7. Multichannel probe according to one of claims 5 or 6, characterized in that the two small channels (16, 18) have a diameter which is equal to about one half the diameter of the large channel (20, 22), or of the two large channels (20, 22).

8. Multichannel probe according to one of claims 1 to 7, characterized in that one channel (16), called treatment channel, in particular a small channel, is intended to receive temporarily an optical fiber adapted to receive a laser radiation, and in particular a radiation from a pulse laser.

9. Multichannel probe according to one of claims 1 to 8, characterized in that one (22) of said channels called observation channel, in particular a large channel, is intended to receive temporarily an optical fiber adapted to receive a light radiation for observing and illuminating the zone outside the tubular element in the vicinity of the distal end.

10. Multichannel probe according to one of claims 1 to 8, characterized in that one channel (18), called irrigation channel, in particular a small channel, receives a irrigation liquid medium for irrigating the zone outside the tubular element in the vicinity of the distal end.

11. Multichannel probe according to one of claims 1 to 10, characterized in that said multichannel probe (10) comprises four independent channels (16, 18, 20, 22) by virtue of three internal partitions (24, 26, 28) disposed longitudinally and substantially parallel to the axis of symmetry of said probe, a first treatment channel (16) for temporarily receiving an optical fiber adapted to receive a laser radiation, in particular a radiation from a pulse laser, a second observation channel (22) for receiving a optical fiber adapted to enable at least the outside zone in the vicinity of the distal end to be observed, a third irrigation channel (18) for receiving a irrigation liquid medium for irrigating the zone outside the distal end, and a fourth insertion channel (20) for receiving a guide wire or leader for facilitating the insertion by guiding of the multichannel probe into a lumen of the body.

12. Multichannel probe according to one of claims 1 to 11, characterized in that said proximal end (14) is provided with a liquid-tight device (30) for coupling the independent channels, the coupling including supply or insertion ducts (32, 34, 36, 38) which are optionally removable or detachable and which enable devices for insertion into the respective channels to be applied thereto and which also enable a irrigation liquid medium to be injected therein.

13. Multichannel probe according to one of claims 1 to 12, characterized in that the tubular element (12) is made, at least partly, from a radio-opaque material.

14. Use of a multichannel probe according to one of claims 1 to 13, for producing an apparatus for destroying lithiasic obstructions by shock waves, notably by means of a pulse laser, in particular a dye pulse laser.

15. Use of a multichannel probe according to one of claims 1 to 13, for producing an apparatus for treating inorganic deposits in ducts, for example under hostile conditions such as within irradiated structures, sludges inside mechanical units, inorganic deposits in steam generator tubes of power stations.

16. Use of a multichannel probe according to one of claims 1 to 13, for producing an apparatus for performing percutaneous surgery of the kidney.

17. Method of endoluminal treatment of inorganic deposits in the ducts containing said inorganic deposits, including the use of a endoluminal probe comprising a hollow tubular element having a proximal end and a treatment distal end, characterized in that a totally hollow tubular element is provided which is subdivided into at least four independent longitudinal channels by at least three internal partitions disposed longitudinally and substantially parallel to the axis of symmetry of the tubular element and extending from the proximal end to the distal end, the external surface of the tubular element being cylindrical and smooth approximately through the whole of its length,
- at least one optical fiber called treatment fiber, of a size adapted to be inserted into at least one of said channels and capable of receiving a beam of laser radiation, in particular radiation from a pulse laser,
- at least one optical fiber called observation fiber, of a size adapted to be inserted in at least another channel and adapted to receive light radiation enabling a zone outside the tubular element and in the vicinity of the distal end to be observed.
- means for injecting at least one irrigation liquid medium into at least one remaining channel.
- said multichannel probe is inserted into a lumen of the duct in which an inorganic deposit is assumed to exist until said distal end comes into contact with said inorganic deposit,
- the optical fiber adapted to receive laser radiation is inserted into its associated channel,
- the optical fiber adapted to transmit observation light radiation is inserted into its associated channel,
- when so desired, an irrigation liquid medium is injected into the irrigation channel by said means for injecting the irrigation liquid.
- the inorganic deposit is destroyed by using laser radiation transmitted by the optical fiber adapted to receive the laser radiation, under visual monitoring of the treatment zone outside the vicinity of the distal end by means of the light radiation transmitted by the observation optical fiber,
- at the end of the inorganic deposit destruction treatment, the treatment optical fiber receiving the laser radiation and the observation optical fiber are withdrawn, and injection of the irrigation liquid medium is stopped, excluding any therapeutic and surgical application of the method.

## Patentansprüche

1. Mehrkanalsonde (10), bestehend aus einem ein distales und ein proximales Ende (13 bzw. 14) aufweisenden Hohlrohr (12), das Hohlrohr bestehend aus einem selbsttragenden, biege-und verwindungsfähigen Material, das durchgehend hohl ausgebildete Rohr (12) ist in mindestens vier voneinander unabhängige Längskanäle (16, 18, 20, 22) durch mindestens drei in Längsrichtung und im wesentlichen parallel zur Symmetrieachse des Hohlrohres (12) vom proximalen Ende zum distalen Ende (13) verlaufende Trennwände (24, 26, 28) unterteilt; die Außenfläche des Hohlrohres ist zylindrisch sowie im wesentlichen über die gesamte Länge glatt.

2. Mehrkanalsonde nach Anspruch 1, **gekennzeichnet durch** deren Anwendung für endocavitäre diagnostische Zwecke und/oder zur endoskopischen Therapie von lithisiatischen Fremdkörpern in der Urologie und Gastroenterologie.

3. Mehrkanalsonde nach Anspruch 1 oder 2, **dadurch gekennzeichnet**, **daß** diese Sonde ein Einweggerät ist.

4. Mehrkanalsonde nach einem der Ansprüche 1 bis 3, **dadurch gekennzeichnet**, **daß** das distale Ende (13) des Hohlrohres (12) aufgeweitet abgeflacht ist.

5. Mehrkanalsonde nach einem der Ansprüche 1 bis 4, **dadurch gekennzeichnet, daß** zwei Kanäle (16, 18), als kleine Kanäle bezeichnet, einen kleineren Durchmesser als die beiden, als große Kanäle bezeichneten Kanäle (20, 22) aufweisen und daß die Anordnung der Trennwände (24, 26, 28) Vorzugsweise einen symmetrischen Aufbau der Sonde in mindestens einer Symmetrieebene (24) gewährleistet.

6. Mehrkanalsonde nach einem der Ansprüche 1 bis 5, **dadurch gekennzeichnet, daß** eine im Durchmesser liegende Trennwand (24) das Hohlrohr (12) gänzlich durchsetzt und daß zwei Trennwände (26, 28) sich von dieser diametralen Trennwand (24) erstrecken und die beiden genannten kleinen Kanäle (16, 18) bilden.

7. Mehrkanalsonde nach einem der Ansprüche 5 oder 6, **dadurch gekennzeichnet, daß** der Durchmesser der beiden kleinen Kanäle (16, 18) etwa die Hälfte des Durchmessers des großen oder der beiden großen Kanäle (20, 22) besitzt.

8. Mehrkanalsonde nach einem der Ansprüche 1 bis 7, **dadurch gekennzeichnet, daß** einer der Kanäle (22), insbesondere ein kleiner Kanal, als ein sogenannter Behandlungskanal der kurzzeitigen Aufnahme einer optischen Faser für eine Laserstrahlung, insbesondere eines gepulsten Lasers, dient.

9. Mehrkanalsonde nach einem der Ansprüche 1 bis 8, **dadurch gekennzeichnet, daß** ein Kanal (22), insbesondere ein großer Kanal, als ein Untersuchungskanal der kurzzeitigen Aufnahme einer Optikfaser für die Weiterleitung eines Lichtstrahls zum Zwecke der Untersuchung und der Beleuchtung des Umfeldes des Hohlrohres im Bereich des distalen Endes dient.

10. Mehrkanalsonde nach einem der Ansprüche 1 bis 8, **dadurch gekennzeichnet, daß** vorzugsweise ein kleiner Kanal als ein sogenannter Einschwemmkanal (18) der Aufnahme eines flüssigen Mediums dient, zum Ausbringen des Mediums in das äußere Umfeld des Hohlrohres im Bereich des distalen Endes.

11. Mehrkanalsonde nach einem der Ansprüche 1 bis 10, **dadurch gekennzeichnet, daß** die Mehrkanalsonde (10) vier voneinander unabhängige Kanäle (16, 18, 20, 22) aufweist, die mittels dreier in Längsrichtung und im wesentlichen parallel zur Symmetrieachse der Sonde verlaufende Trennwände (24, 26, 28) getrennt sind, wobei ein erster Behandlungskanal (16) kurzzeitig eine Optikfaser für eine Laserstrahlung aufnimmt, vorzugsweise eines gepulsten Lasers, während ein zweiter Untersuchungskanal (22) zur Aufnahme einer Optikfaser für zumindest die Beobachtung des Umfeldes am distalen Ende dient und ein dritter Einschwemmkanal (18) ein flüssiges Medium zur Ausbringung im äußeren Bereich des distalen Endes aufnimmt und daß ein vierter Einführkanal (20) einen das Einführen der Mehrkanalsonde in einen Körperhohlraum erleichternden Führungsleiter oder "leader" aufnimmt.

12. Mehrkanalsonde nach einem der Ansprüche 1 bis 11, **dadurch gekennzeichnet, daß** am proximalen Ende (14) ein abdichtendes, gegebenenfalls abnehmbares oder abtrennbares Anschlußstück (30) für die separaten Kanäle vorgesehen ist, das Versorgungs- oder Einführleitungen aufweist, über welche die jeweils in die Kanäle einzusetzenden Instrumente oder das zu injizierende flüssige Medium einführbar ist.

13. Mehrkanalsonde nach einem der Ansprüche 1 bis 12, **dadurch gekennzeichnet, daß** das Hohlrohr zumindest teilweise aus einem röntgenstrahlen-undurchlässigen Material besteht.

14. Verwendung einer Mehrkanalsonde nach einem der Ansprüche 1 bis 13 als Teil eines Gerätes zur Zerstörung von lithiasischen Fremdkörpern durch Stoßwellen, insbesondere durch gepulste Laserstrahlen, vorzugsweise durch einen Farblaser.

15. Verwendung einer Mehrkanalsonde nach einem der Ansprüche 1 bis 13 als Teil eines Gerätes zur Behandlung von mineralischen Ablagerungen in Rorleitungen, insbesondere an ungünstigen nicht unmittelbar zugänglichen Stellen, zur Behandlung von Ablagerungen innerhalb von mechanisch wirkenden Bau-Elementen und zur Behandlung von Mineralstoffablagerungen in Rohrleitungen von Dampferzeugern einer zentralen Wärmeerzeugungs-Einrichtung.

16. Verwendung einer Mehrkanalsonde nach einem der Ansprüche 1 bis 13 als Teil eines Gerätes für invasive Eingriffe an der menschlichen Niere.

17. Verfahren zur Behandlung von Mineralstoffablagerungen in Hohlleitungen im Wege der Einführung einer endoluminalen, aus einem ein distales und ein proximales Ende aufweisenden Hohlrohr bestehenden Sonde in Körperhohlräume, **gekennzeichnet durch** die Verwendung eines selbsttragenden, biege- und verwindungsfähigen Materials für ein durchgehend hohl ausgebildetes Rohr, das in mindestens vier voneinander getrennte Längskanäle durch mindestens drei in Längsrichtung und im wesentlichen symmetrisch zur Symmetrieachse des Hohlrohres verlaufende Trennwände unterteilt ist, wobei die Außenfläche des Hohlrohres zylindrisch und im wesentlichen über die gesamte Länge glatt ist, ferner **gekennzeichnet durch**
- die Verwendung mindestens einer Optikfaser als Behandlungsfaser mit einem den Abmessungen mindestens eines der Kanäle zwecks Einführung angepaßten Durchmessers zur Aufnahme eines gebündelten Laserstrahls, insbesondere eines gepulsten Lasers,
- die Verwendung mindestens einer Optikfaser als Untersuchungsfaser mit einem den Abmessungen mindestens eines weiteren Kanals zwecks Einführung angepaßten Durchmessers zur Weiterleitung von Lichtstrahlen, die das Ausleuchen des Umfeldes des Hohlrohres im Bereich von dessen distalem Ende ermöglicht,
- Einführen eines Mittels zur Injektion, mindestens eines flüssigen Ausschwemmediums in mindestens einem der verbleibenden Kanäle,
- Einführen der Mehrkanalsonde in den Körperhohlraum eines Gefäßes, in dem eine Mineralstoffablagerung vermutet wird, bis das distale Ende an der Mineralstoffablagerung auftrifft,
- Einführen der Optikfaser für die Anwendung der Laserstrahlung in ihren zugeordneten Kanal,
- Einführen der Optikfaser zur Übertragung eines Lichtstrahls zum Zwecke der Untersuchung in den zugehörigen Kanal,
- bei Bedarf Einspritzen eines flüssigen Ausschwemmmediums in den Ausschwemmkanal vermittels einer Einspritzvorrichtung,
- Zerstören der Mineralstoffablagerung mit Hilfe von Laserstrahlen, die durch die hierzu ausgelegte Optikfaser übermittelt werden unter visueller Kontrolle der Untersuchung des äußeren Umfeldes am distalen Ende mit Hilfe der Lichtstrahlen, die über die Untersuchungs-Optikfaser einfallen,
- sowie Abschluß des Zertrümmerungsvorgangs der Mineralstoffablagerung durch Entfernen der Untersuchungs-Optikfaser und Abstellen der Injektion von Ausschwemmflüssigkeit ohne Beendigung einer therapeutischen Behandlung und eines chirurgischen Eingriffs.
